Europäisches Patentamt

European Patent Office

Office européen des brevets

(19)

(11) Veröffentlichungsnummer : **0 071 025**
**B1**

(12) EUROPÄISCHE PATENTSCHRIFT

(45) Veröffentlichungstag der Patentschrift :
13.03.85

(21) Anmeldenummer : 82105688.4

(22) Anmeldetag : 26.06.82

(51) Int. Cl.⁴ : **C 07 C 57/02**, C 07 C 51/41,
A 61 K 31/19, A 61 K 7/36,
A 61 K 7/06, A 61 K 7/48

(54) Zinksalz der hydrolisierten Tricarbonsäure aus En-Addukten von Maleinsäureanhydrid an Undecylensäure, Verfahren zur Herstellung derselben und ihre Verwendung.

(30) Priorität : 29.07.81 DE 3129826

(43) Veröffentlichungstag der Anmeldung :
09.02.83 Patentblatt 83/06

(45) Bekanntmachung des Hinweises auf die Patenterteilung : 13.03.85 Patentblatt 85/11

(84) Benannte Vertragsstaaten :
CH FR GB IT LI NL

(56) Entgegenhaltungen :
EP-A- 0 023 676
EP-A- 0 046 970
DE-A- 2 460 205
FR-A- 2 125 219
US-A- 2 569 420
CHEMICAL ABSTRACTS, Band 66, Nr. 13, 27. März 1967, Seite 6106, Nr. 65108y, Columbus, Ohio, USA

(73) Patentinhaber : Grillo-Werke AG
Postfach 11 01 09
D-4100 Duisburg-Hamborn (DE)

(72) Erfinder : Lowicki, Norbert, Dr. Ing. Dipl.-Chem.
Walramsweg 26
D-4100 Duisburg 1 (DE)
Erfinder : Desai, Natvarlal B., Dr. rer.nat. Dipl.-Chem.
Margaretenweg 35
D-4220 Dinslaken (DE)

(74) Vertreter : Werner, Hans-Karsten, Dr. et al
Deichmannhaus am Hauptbahnhof
D-5000 Köln 1 (DE)

**0 071 025**

## Beschreibung

Die Erfindung betrifft Zinksalze der hydrolisierten Tricarbonsäure aus En-Addukten von Maleinsäureanhydrid an Undecylensäure, Verfahren zu ihrer Herstellung und ihre Verwendung als hautfreundliche bakterizide und fungizide Mittel, insbesondere in kosmetischen Präparaten.

En-Addukte bzw. Diels-Alder-Addukte vom Maleinsäureanhydrid mit mindestens einfach ungesättigten Carbonsäuren sind bekannt. Sie können mit Alkali verseift werden und ihre Ester und Amide, insbesondere ihre Umsetzungsprodukte mit Triäthanolamin sind als Korrosionsschutzmittel bekannt geworden ; vergl. US-PS 3 985 504.

Die Alkalisalze sind auch schon zur Verhinderung von Ablagerungen von heißem Meereswasser verwendet worden ; vgl. GB-PS 1 551 894. Die chemische Struktur der Addukte schien längere Zeit noch nicht völlig geklärt zu sein, da sich die Angaben hierüber in den beiden oben genannten Patentschriften widersprechen. Gemäß US-PS 3 985 504 findet eine Anlagerung von Kohlenstoffatomen neben der Doppelbindung statt, wobei die Doppelbindung erhalten bleibt. Gemäß GB-PS 1 551 894 entstehen Cyclobutandicarbonsäuren. Auf alle Fälle entstehen durch Hydrolyse der Addukte drei- und mehrwertige Carbonsäuren.

Inzwischen wurde herausgefunden, daß Maleinsäureanhydrid mit einfach ungesättigten Carbonsäuren Addukte bildet, bei denen ein Kohlenstoffatom in der Doppelbindung des Maleinsäureanhydrids mit einem Kohlenstoffatom der Doppelbindung der Carbonsäure eine neue Kohlenstoff-Kohlenstoff-Bindung bildet. Das zweite Kohlenstoffatom der Doppelbindung des Maleinsäureanhydrids verwandelt sich dabei in eine aktive $CH_2$-Gruppe. Die Doppelbindung der Carbonsäure bleibt zwar erhalten, jedoch um ein Kohlenstoffatom zur Carboxylgruppe hin verschoben. Diese Reaktion wird nach neuerer Nomenklatur En-Reaktiongenannt, die Addukte nennt man jetzt En-Addukte. Diese Reaktion wurde erstmals 1962 beschrieben in Liebig's Annalen der Chemie, 651, Seite 141.

Es ist bereits vorgeschlagen worden, Partialester von zwei- und mehrwertigen Alkoholen, insbesondere aber auch von Mono- und Disacchariden mit den Diels-Alder- bzw. En-Addukten von Maleinsäureanhydrid an mindestens einfach ungesättigte Carbonsäuren mit einer Kettenlänge von $C_{10}$ bis $C_{25}$ als hautfreundliche, nicht ionogene oberflächenaktive Substanzen herzustellen und zu verwenden. Die gewünschtenfalls sulfierten Partialester der Undecylensäure werden als hautfreundliche, mild bakterizide und fungizide nicht ionogene und/oder anionenaktive oberflächenaktive Substanzen in kosmetischen Präparaten genannt ; vgl. deutsche Patentanmeldung P 30 32 612.6 entsprechend EP-A-0 046 970.

Es wurde jetzt gefunden, daß die Zinksalze der hydrolisierten Tricarbonsäuren aus En-Addukten von Maleinsäureanhydrid an Undecylensäure überraschend hohe bakterizide und fungizide Wirkungen aufweisen, dabei jedoch sehr hautfreundlich und verträglich sind. Sie eignen sich daher in besonderem Maße zur Verwendung in Körperpflegemitteln, bei denen eine hautschonende biozide Wirksamkeit besonders erwünscht ist, beispielsweise in Intim- und Fußpflegemitteln sowie antischuppenwirksamen Haarshampoos.

Die erfindungsgemäßen Zinksalze der hydrolisierten Tricarbonsäuren aus En-Addukten von Maleinsäureanhydrid an Undecylensäure sind dabei allen bisher bekannten und für diesen Zweck verwendeten Substanzen bezüglich ihrer Wirksamkeit und ihrer Verträglichkeit erheblich überlegen. Diese Eigenschaften machen sie obendrein geeignet als Konservierungsmittel in kosmetischen Präparaten verwendet zu werden, da von ihnen keinerlei physiologisch schädigende Wirkungen zu erwarten sind.

Die erfindungsgemäßen Zinksalze weisen je nach den Herstellungsbedingungen eine gewisse Mengen an freien Carbonsäuregruppen auf. Das Molverhältnis Zink zu Tricarbonsäure beträgt somit im allgemeinen 0,8 bis 1,2. Erfindungsgemäß sind aber auch die Zinksalze mit einem höheren und einem niedrigeren Zinkgehalt herstellbar und verwendbar. Die Herstellung der Zinksalze erfolgt am einfachsten dadurch, daß man die En-Addukte vom Maleinsäureanhydrid mit Undecylensäure mit wässrigem Alkali hydrolisiert und anschließend mit einem wasserlöslichen Zinksalz umsetzt. Die Umsetzung kann prinzipiell im gesamten Temperaturbereich zwischen 0 und 100 °C erfolgen, jedoch werden Temperaturen zwischen 50 und 80 °C bevorzugt.

Das Zinksalz wird dabei in etwa äquimolaren Mengen zugesetzt, vorzugsweise im Bereich zwischen 0,9 und 1,3 Mol pro Mol Tricarbonsäure. Um Salze mit höherem oder niedrigerem Zinkgehalt zu erhalten, können selbstverständlich auch höhere oder niedrigere Mengen Zinksalz eingesetzt werden. Bei den oben genannten Herstellungsbedingungen fällt ein Zinksalz aus, welches etwa 1 Mol Zink auf 1 Mol Tricarbonsäure enthält und einen pH-Wert von 5,5 bis 5,7 aufweist.

Die bekannte antimikrobielle Wirksamkeit der Salze der Undecylensäure, insbesondere deren Zinksalz hat nur zu begrenzter Anwendung derselben in der Haarkosmetik geführt. Für diese Salze, die auch für Verbindungen, die in größerem Umfang in der Haarkosmetik als Antischuppenmittel Verwendung finden (Selendisulfid, Zinkpyrithion), gilt offenbar übereinstimmend, daß ihre äußerst geringe Löslichkeit bzw. ihre mangelnde Substantivität gegenüber Kopfhaut und Haaren ihre Wirkung nur auf den unmittelbaren Haarwasch-Vorgang begrenzt.

Die überraschend hohe Wirksamkeit der erfindungsgemäßen Zinkverbindung im Vergleich zu den bekannten, die zum Teil in Vitro höhere antimikrobielle bzw. fungizide Wirksamkeit besitzen, beruht offenbar zu einem wesentlichen Teil auf dem Vorhandensein einer freien Carboxylgruppe, wodurch die

Substantivität diese; Verbindung für Haar und Kopfhaut signifikant erhöht wird. Ein weiterer positiver Aspekt dieser Carboxylgruppe liegt darin, daß die Verbindung schwach sauer reagiert und dadurch die Biosphäre der Kopfhaut für Pilze, speziell vom Typ Pythirosporum, denaturiert wird.

Die Antischuppenwirkung der erfindungsgemäßen Zinkverbindung übertrifft deutlich diejenige z. B. von Zinkpyrithion und dies offenbar dadurch, weil die neue Zinkverbindung stärker und länger anhaltend im gewünschten Sinn Kopfhaut und Haare beeinflußt.

In den nachfolgenden Beispielen wird die Erfindung näher erläutert.

## Beispiel 1

184,3 g Undecylensäure (1 Mol) werden mit 98 g Maleinsäureanhydrid (1 Mol) ohne Lösungsmittel in einem Dreihalskolben unter Rühren in Stickstoffatmosphäre bei 170 bis 175 °C innerhalb von 5 Stunden umgesetzt. Nach Beendigung der Reaktion wird das Reaktionsprodukt auf 60 bis 70 °C abgekühlt und mit wässriger NaOH hydrolisiert. Anschließend gibt man 287,46 g $ZnSO_4 \cdot 7H_2O$, gelöst in 1 000 ml Wasser, bei 70 °C zu. Das ausgefallene Zinksalz wird mehrfach mit destilliertem Wasser ausgewaschen und bei 70 °C getrocknet. Die Ausbeute beträgt 290 g. Die Analyse ergab 17,52 % Zink. Bei einem Molverhältnis 1 : 1 errechnet sich theoretisch 17,97 % Zink.

## Beispiel 2

Antischuppenshampoo

| | |
|---|---:|
| Zinksalz gemäß Beispiel 1 | 2,0 % |
| Natriumlauryläthersulfat (28 %) | 34,0 % |
| Kokosfettsäurediäthanolamid | 2,5 % |
| Natriumchlorid | 2,0 % |
| Wasser | 59,5 % |
| | 100,0 % |

## Beispiel 3

Intimpflege-Waschlösung

| | |
|---|---:|
| Zinksalz gemäß Beispiel 1 | 0,5 % |
| Sulfitierter Partialester von Äthylenglykol des Diels-Alder-Adduktes von Maleinsäureanhydrid mit Undecylensäure (gemäß Beispiel 5 der deutschen Patentanmeldung P 30 32 612.6 entsprechend US- Application Serial No 240 606) | 10,0 % |
| Ricinolsäuremonoäthanolamidsulfosuccinat | 15,0 % |
| Natriumlauryläthersulfat (28 %) | 20,0 % |
| Comperlan KD (Kokofettsäurediäthanolamid — Henkel KGaA) | 3,0 % |
| Polyglykol 400 | 10,0 % |
| Polyol-Fettsäureester | 7,0 % |
| Wasser | 34,5 % |
| | 100,0 % |

## Beispiel 4

Fußpuder — antimykotisch

| | |
|---|---:|
| Talkum | 72,0 % |
| China-Clay | 20,0 % |
| Reisstärke | 5,2 % |
| Zinksalz gemäß Beispiel 1 | 2,5 % |
| Aerosil 2 000 (pyrogenes Siliciumdioxid) | 0,3 % |
| | 100,0 % |

Beispiel 5

Desodorierende Intimpflegelotion

| | |
|---|---|
| Grillocin Hy 77 (Deowirkstoff Zinkricinoleatbasis Grillo AG) | 1,0 % |
| Cremophor A25 (Fettalkoholpolyglykoläther) | 1,0 % |
| Mono- und Diglyceride der Palmitin- und Stearinsäure | 6,0 % |
| Triglycerid gesättigter Pflanzenfettsäuren | 4,0 % |
| Paraffinöl | 6,0 % |
| Cetylalkohol | 1,0 % |
| 1,2 Propylenglykol | 3,0 % |
| Zinksalz gemäß Beispiel 1 | 0,3 % |
| Wasser | 77,7 % |
| | 100,0 % |

Die hohe antimikrobielle Wirksamkeit der Salze gemäß Beispiel 1 wurde getestet. Die Ergebnisse sind in der nachfolgenden Tabelle 1 zusammengestellt. Die minimalen Hemmkonzentrationen (MHK) finden sich in der Tabelle 2.

Die akute Toxizität der Salze gemäß Beispiel 1 wurde an der Ratte getestet. Die $LD_{50}$ oral wurde dabei sowohl bei männlichen wie bei weiblichen Ratten als größer als 16 000 mg/kg gefunden. Die Toxizität bisher verwendeter Antischuppenmittel beträgt gemäß N. P. Lupke und P. Preusser, Antischuppenkosmetika — Wirkung und Toxikologie, Ärztliche Kosmetologie 8, S. 5, 1978, bei der Ratte ausgedrückt als $LD_{50}$ Werte zwischen 56 und 1120. Im einzelnen betrugen diese Werte bei Hexachlorophen (männlich 66, weiblich 56) ; bei Selendisulfid 138, bei Zinkpyrithion (männlich 200, weiblich 140) und bei Natriumpyrithion (männlich 1.120, weiblich 980). Die erfindungsgemäße Substanz ist somit wesentlich weniger toxisch.

Um die dermale Verträglichkeit der erfindungsgemäßen Zinkverbindung zu prüfen, wurde ein Test auf der intakten und der skarifizierten Haut von Kaninchen durchgeführt. Der Test wurde in Anlehnung an die Angaben der « Consumer Product Safety Commission der USA (Code of Federal Regulations, Title 16, Section 1 500.41) » durchgeführt. Die Bewertung der Hautreaktion wurde entsprechend den ETAD-Empfehlungen vorgenommen. Sowohl nach 24 Std. wie nach 72 Std. wurden weder auf der intakten noch der skarifizierten Haut Erytheme oder Ödeme beobachtet.

Die Überprüfung der subchronischen und chronischen Toxizität bei der Ratte mit Dosen von 15, 375 und 939 ppm im Futter ergab innerhalb von 3 Monaten keinerlei negative Wirkungen, während bei vergleichbaren Dosen von Zink-2-pyridinthiol-1-oxid bei vielen Tieren Lähmung der hinteren Extremitäten und Todesfälle beobachtet wurden. Unverdünntes Shampoo gemäß Beispiel 2 wirkt auf das Kaninchenauge nur leicht reizend, während mit einem Shampoo enthaltend Zink-2-pyridinthiol-1-oxid bei allen getesteten Tieren Corneaschäden beobachtet wurden.

(Siehe Tabellen, Seite 5 ff.)

## Tabelle 1

Antimikrobielle Wirksamkeit der Verbindung nach Beispiel 1

Konzentration : 2 %

| Einwirkungszeit Minuten | Escherichia coli | Staphylococcus aureus | Trichophyton mentagrophytes | Candida albicans | Pityrosporon ovale |
|---|---|---|---|---|---|
| 2,5 | - | - | - | - | - |
| 5,0 | - | - | - | - | - |
| 10,0 | - | - | - | - | - |
| 20,0 | - | - | - | - | - |
| 30,0 | - | - | - | - | - |
| Blindwert | +++ | +++ | + | ++ | + |
| Ausgangskeimgehalt je ml der beimpften Lösung | 280.000 | 120.000 | 24.000 | 9.000 | 440.000 |

Zeichenerklärung :

+++ sehr starkes Wachstum
++ deutliches Wachstum
+ schwaches Wachstum
− kein Wachstum

Tabelle 2

Werte der minimalen Hemmkonzentration (MGK) der Verbindung nach Beispiel 1

| Bestimmungen des MHK durch Verdünnungstest | Escherichia coli | Staphylococcus aureus | Pseudomonas aeruginosa | Candida albicans | Pityrosporon ovale |
|---|---|---|---|---|---|
| Konzentration in % der Originalsubstanz | | | | | |
| 10 % | – | – | – | – | – |
| 5 % | – | – | – | – | – |
| 2,5 % | – | – | – | – | – |
| 1,0 % | – | – | – | – | – |
| 0,5 % | – | – | – | – | – |
| 0,1 % | – | (+) | (+) | – | – |
| 0,05 % | (+) | + | + | (+) | (+) |
| Anzahl der Testkeime | 82.000 | 26.000 | 73.000 | 4.000 | 8.500 |

Zeichenerklärung :

  – kein Wachstum
(+) vereinzelt Wachstum
 + deutliches Wachstum

**Ansprüche**

1. Zinksalze der hydrolisierten Tricarbonsäure aus En-Addukten von Maleinsäureanhydrid an Undecylensäure.

2. Zinksalze gemäß Anspruch 1, dadurch gekennzeichnet, daß das Molverhältnis 0,8 bis 1,2 beträgt.

3. Verfahren zur Herstellung der Zinksalze der hydrolisierten Tricarbonsäuren aus En-Addukten vom Maleinsäureanhydrid an Undecylensäure, dadurch gekennzeichnet, daß man die En-Addukte von Maleinsäureanhydrid an Undecylensäure hydrolisiert und mit wasserlöslichem Zinksalz umsetzt.

4. Verfahren gemäß Anspruch 3, dadurch gekennzeichnet, daß man die Umsetzung mit 0,9 bis 1,5 Mol Zink pro Mol der hydrolisierten Tricarbonsäure durchführt.

5. Verwendung der Zinksalze gemäß Anspruch 1 oder 2 als hautfreundliche, bakterizide und fungizide Mittel in kosmetischen Präparaten.

**Claims**

1. Zinc salts of the hydrolyzed tricarboxylic acid from ene-adducts of maleic anhydride to undecylenic acid.

2. Zinc salts according to claim 1, characterized in that the molar ratio is from 0.8 to 1.2.

3. A process for preparing the zinc salts of the hydrolyzed tricarboxylic acids from ene-adducts of maleic anhydride to undecylenic acid, characterized in that the ene-adducts of maleic anhydride to undecylenic acid are hydrolyzed and reacted with a water-soluble zinc salt.

4. The process according to claim 3, characterized in that the reaction is carried out by using 0.9 to 1.5 moles of zinc per mole of the hydrolyzed tricarboxylic acid.

5. Use of the zinc salts according to claims 1 or 2 as skin-compatible bactericidal and fungicidal agents in cosmetic preparations.

**Revendications**

1. Sels de zinc de triacides carboxyliques hydrolysés, obtenus à partir des produits d'addition éniques de l'anhydride maléique avec l'acide undécylénique.

2. Sels de zinc selon la revendication 1, caractérisés en ce que le rapport molaire est de 0,8 à 1,2.

3. Procédé de préparation des sels de zinc de triacides carboxyliques hydrolysés obtenus à partir des produits d'addition éniques de l'anhydride maléique avec l'acide undécylénique, caractérisé en ce que l'on hydrolyse des produits d'addition éniques de l'anhydride maléique avec l'acide undécylénique et qu'on le fait réagir avec un sel de zinc hydrosoluble.

4. Procédé selon la revendication 3, caractérisé en ce que l'on réalise la réaction avec 0,9 à 1,5 mole par mole de triacide carboxylique hydrolysé.

5. Utilisation des sels de zinc selon la revendication 1 ou 2, comme bactéricides et fongicides non agressifs pour la peau dans des préparations cosmétiques.